# EUROPÄISCHE PATENTSCHRIFT

(11) **EP 2 163 236 B1**
(45) Veröffentlichungstag und Bekanntmachung des Hinweises auf die Patenterteilung: **01.05.2013**
(21) Anmeldenummer: 09166972.1
(22) Anmeldetag: 31.07.2009
(51) Int. Cl.: A61K 8/14, A61K 8/55, A61K 8/04, A61Q 19/08

(54) **Kosmetische Basiszusammensetzung umfassend Hüllliposomen, die drei oder vier Liposomen mit unterschiedlichen kosmetischen Wirkstoffen einschließen und Verfahren zur Herstellung**
Cosmetic base composition comprising liposomes which incorporate three or four liposomes with different cosmetic agents and production method
Composition de base cosmétique comprenant des liposomes à enveloppe, lesquels comprennent trois ou quatre liposomes ayant des effets cosmétiques différents et procédé de fabrication

(30) Priorität: 31.07.2008 DE 102008035834
(43) Veröffentlichungstag der Anmeldung: 17.03.2010
(73) Patentinhaber: Coty Germany GmbH, 55116 Mainz (DE)
(72) Erfinder: Golz-Berner, Karin, 98000, Monaco (MC); Zastrow, Leonhard, 98000, Monaco (MC)
(74) Vertreter: Rasch, Dorit

(56) Entgegenhaltungen:
- EP-A1- 0 661 037
- WO-A1-2008/003638
- CH-A5- 678 489
- GARCIA-ANTON, J. M. ET AL: "Plurilamellar multivesicular liposomes: Methodology and cosmetic applications", SOFW JOURNAL, 122(4), 206, 208-9 CODEN: SOFJEE; ISSN: 0942-7694, 1996, XP009142899,
- GARCIA-ANTON, J. M.: "The Scientific Response to different types of wrinkles", SOFW JOURNAL , 132(4), 8, 10, 12-14, 16 CODEN: SOFJEE; ISSN: 0942-7694, 2006, XP009142896,
- DATABASE CA [Online] CHEMICAL ABSTRACTS SERVICE, COLUMBUS, OHIO, US; 7. August 2006 (2006-08-07), KAHNE, ZDENKA: "Cosmetic composition and procedure for depth reduction of skin wrinkles", XP002615419, gefunden im STN Database accession no. 2006:776774 -& SI 21 911 A (KOZMETICNIH STORITEV TRGOVINA, SLOVENIA) 30. Juni 2006 (2006-06-30)
- YAMABE K ET AL: "In vitro characteristics of liposomes and double liposomes prepared using a novel glass beads method", JOURNAL OF CONTROLLED RELEASE, ELSEVIER, AMSTERDAM, NL, Bd. 90, Nr. 1, 5. Juni 2003 (2003-06-05), Seiten 71-79, XP004427407, ISSN: 0168-3659, DOI: DOI:10.1016/S0168-3659(03)00159-7
- KATAYAMA, KEN ET AL: "Preparation of novel double liposomes using the glass-filter method", INTERNATIONAL JOURNAL OF PHARMACEUTICS ( 2002 ), 248(1-2), 93-99 CODEN: IJPHDE; ISSN: 0378-5173, 2002, XP002615420,
- KATAYAMA, KEN ET AL: "Double Liposomes: Hypoglycemic Effects of Liposomal Insulin on Normal Rats", DRUG DEVELOPMENT AND INDUSTRIAL PHARMACY ( 2003 ), 29(7), 725-731 CODEN: DDIPD8; ISSN: 0363-9045, 2003, XP002615421,

## Beschreibung

Die Erfindung betrifft ein Verfahren zur Herstellung einer kosmetischen Basiszusammensetzung umfassend Hüllliposomen, die drei oder vier Liposomen mit unterschiedlichen kosmetischen Wirkstoffen einschließen und in einer Gelbasis eingebettet sind. Die Erfindung betrifft weiterhin eine nach dem Verfahren hergestellte kosmetische Basiszusammensetzung und kosmetische Zubereitungen, die die kosmetische Basiszusammensetzung umfassen.

Liposomen werden in der Medizin und Kosmetik als intrakorporales oder extrakorporales Transportsystem für verschiedene Aktiv- oder Wirkstoffe verwendet, insbesondere für die dermale und transdermale Applikation von Wirkstoffen. Die Liposomen sind kugelförmige Vesikel, die durch Lipide gebildet werden, deren hydrophile und hydrophobe Eigenschaften zur Ausbildung einer Doppelschicht führen und dabei einen wasserhaltigen Hohlraum umschließen, der für Transportzwecke genutzt werden kann. Der Aufbau und die Herstellung verschiedenartiger unilamellarer und multilamellarer Liposomen ist bekannt und in vielen Veröffentlichungen beschrieben.

Da die Stabilität der Liposomen bei der topischen Anwendung und entsprechender mechanischer Belastung nicht immer lange anhält, werden die Wirkstoffe oftmals frühzeitiger oder schneller als erwünscht freigesetzt. Andererseits besteht bei Liposomen mit größeren Wandstärken z.B. durch eine Vielzahl von multilamellaren konzentrischen Lipiddoppelschichten das Problem, dass ihre Stabilität zu hoch ist und eine Freigabe der Wirkstoffe deutlich schwieriger zu erreichen ist. In beiden Fällen sind hohe Liposomkonzentrationen erforderlich, um ein angestrebtes Niveau an Wirkstoffkonzentration in der Haut zu realisieren. Bei gleichzeitigem Einsatz verschiedenartiger liposomal verkapselter Wirkstoffe bestehen diese Probleme in erhöhtem Maße.

Der Erfindung liegt die Aufgabe zugrunde, ein Herstellungsverfahren für kosmetische Basiszusammensetzungen mit unterschiedlichen Wirkstoffen bereitzustellen.

Eine weitere Aufgabe besteht darin, kosmetische Basiszusammensetzungen auf Liposomenbasis mit unterschiedlichen Wirkstoffen zu entwickeln und dabei die Stabilität der Zusammensetzungen für einen ausreichend langen Zeitraum zu gewährleisten.

Eine weitere Aufgabe besteht darin, ein kosmetisches Mittel bereitzustellen, das mehrere Wirkstoffe zur Verbesserung der Hautregenerierung und Anti-Alterungswirkung aufweist und das bei niedriger Wirkstoffkonzentration eine verbesserte Langzeitwirkung zeigt.

Erfindungsgemäß wird ein Verfahren zur Herstellung einer kosmetischen Basiszusammensetzung, die Hüllliposomen mit einer Partikelgröße von 250 - 600 nm in einem wässrigen Gel mit einer Viskosität im Bereich von 4.000 bis 20.000 mPa·s umfasst, die in ihrem wässrigen Volumen drei oder vier Liposomen einschließen, die jeweils mindestens einen Wirkstoff in ihrem wässrigen Volumen enthalten, wobei die in den eingeschlossenen Liposomen enthaltenen Wirkstoffe voneinander verschieden sind und die eingeschlossenen Liposomen eine Partikelgröße im Bereich von 50 - 200 nm haben, bereitgestellt, welches dadurch gekennzeichnet ist, dass die drei oder vier Liposomen unter Rühren in Wasser eingebracht werden und anschließend in das Liposom-Wasser-Gemisch nacheinander ein Liposombildner, ein Gelbildner und ein Neutralisierungsmittel eingebracht werden.

Dabei werden die wenigstens zwei Liposomen nacheinander unter Rühren mit 100-150 U/min in einem Zeitraum von 10 bis 20 Minuten in Wasser eingebracht. Danach wird ein flüssiger Liposombildner unter Rühren mit 50-90 U/min mit einer Geschwindigkeit von 1,5-3,5 l/h in das Liposom-Wasser-Gemisch eingebracht, wobei die Temperatur während dieser Zeit zwischen 15 und 40 °C gehalten wird. Danach wird ein Gelbildner unter Rühren mit 50-90 U/min über einen Zeitraum von 20-50 Minuten bis zum Erreichen eines homogenen Gemisches einbracht. Anschließend wird unter Rühren bei der gleichen Rührgeschwindigkeit ein Neutralisierungsmittel zugegeben und dann wird die Rührgeschwindigkeit für 30-60 Minuten auf 500-1.000 U/min erhöht, wobei dabei die Temperatur unter 40 °C gehalten wird, wobei als Liposombildner Phospholipide eingesetzt werden.

In einer bevorzugten Ausführungsform des erfindungsgemäßen Verfahrens werden die Liposomen nacheinander unter Rühren mit 110-130 U/min in einem Zeitraum von 10 bis 20 Minuten in das Wasser eingebracht. Das Wasser weist vorzugsweise eine Temperatur im Bereich von 15 bis 40 °C, noch bevorzugter im Bereich von 25 bis 40 °C auf. Der flüssige Liposombildner wird vorzugsweise unter Rühren mit 60-80 U/min, vorzugsweise mit einer Geschwindigkeit von 2-3 l/h in das Liposom-Wasser-Gemisch eingebracht. Die Temperatur wird während dieser Zeit im Bereich von 15 bis 40 °C, vorzugsweise im Bereich von 25 bis 40 °C gehalten. Der Gelbildner wird bevorzugt unter Rühren mit 60-80 U/min über einen Zeitraum von 20-50 Minuten bis zum Erreichen eines homogenen Gemisches einbracht. Nach Zugabe des Neutralisierungsmittels wird die Rührgeschwindigkeit für 30-60 Minuten vorzugsweise auf 600 - 800 U/min erhöht. Halten der Temperatur unter 40 °C bedeutet erfindungsgemäß, dass man bei Umgebungs-temperatur (15-25°C) oder darüber arbeiten kann, nicht jedoch höher als 40 °C.

Das erfindungsgemäße Verfahren ist besonders durch die drei Rührgeschwindigkeiten, die Zugabereihenfolge und die Zugabegeschwindigkeiten gekennzeichnet. Ferner müssen bestimmte Temperaturbereiche eingehalten werden. Die initiale Rührgeschwindigkeit ist derart ausgelegt, dass die Liposomen gleichmäßig im Wasser verteilt werden und ihre Integrität erhalten bleibt. Anschließend wird die Rührgeschwindigkeit gesenkt um, die größeren Hüllliposomen zu bilden und diese in das Gel einzuarbeiten. Die finale Erhöhung der Rührgeschwindigkeit führt zu einer besonders stabilen und äußerst homogenen kosmetischen Basiszusammensetzung.

Es wurde gefunden, dass mit dem erfindungsgemäßen Verfahren Liposomen mit verschiedenen Wirkstoffen gemeinsam in ein größeres Hüllliposom eingeschlossen werden können, und dieses größere Hüllliposom stabil gehalten werden kann und seine Stabilität auch durch nachfolgende Mischprozesse zur Einarbeitung in Emulsionen oder Gele nicht verliert. Beim Auftragen derartiger Emulsionen oder Gele auf die Haut bleiben die größeren Hüllliposomen mit den darin eingeschlossenen kleineren Liposomen weitgehend erhalten und werden erst innerhalb des Hautgewebes zusammen mit den dann freigegebenen kleineren Liposomen abgebaut. Dadurch kommt es zu einer sehr konzentrierten gemeinsamen Wirkung der aus den kleineren Liposomen freigesetzten verschiedenen Wirkstoffe, wobei diese Wirkung sowohl sehr schnell auftritt als auch sehr lange anhält.

Die Erfindung betrifft ferner die kosmetische Basiszusammensetzung die mittels des erfindungsgemäßen Verfahrens erhältlich ist und erhalten wird. Die Erfindung betrifft also eine kosmetische Basiszusammensetzung umfassend Hüllliposomen, wobei die Hüllliposomen in ihrem wässrigen Volumen wenigstens zwei Liposomen einschließen, die jeweils mindestens einen Wirkstoff in ihrem wässrigen Volumen enthalten, wobei die in den eingeschlossenen Liposomen enthaltenen Wirkstoffe voneinander verschieden sind und die eingeschlossenen Liposomen eine Partikelgröße im Bereich von 50-200 nm haben, und die Hüllliposomen eine Partikelgröße von 250-600 nm haben und die Hüllliposomen in einem wässrigen Gel mit einer Viskosität im Bereich von 4.000 bis 20.000 mPa·s vorliegen.

Vorzugsweise enthält das wässrige Gel, in dem die Hüllliposomen verteilt sind, eine schwache Base als Neutralisierungsmittel.

Vorteilhafte Ausführungsformen des erfindungsgemäßen Verfahrens und der erfindungsgemäßen kosmetischen Basiszusammensetzung sind in den Unteransprüchen und im Folgenden aufgeführt. Alle vorteilhaften Eigenschaften und Ausführungsformen, die im Zusammenhang mit dem erfindungsgemäßen Verfahren beschrieben werden, gelten analog für die Eigenschaften und Ausführungsformen der mit diesem Verfahren hergestellten kosmetischen Basiszusammensetzungen selbst und umgekehrt.

In einer bevorzugten Ausführungsform des erfindungsgemäßen Verfahrens wird das Rühren ohne Verwirbelung durchgeführt.

Als Neutralisierungsmittel ist erfindungsgemäß eine schwache Base, vorzugsweise eine schwache organische Base geeignet. Geeignete organische Basen sind Mono-, Di- oder Triethanolamin, Trimethylamin, Triethylamin, Tripropylamin, Tributylamin, Imidazol oder Pyridin. Besonders bevorzugt wird Triethylamin als Neutralisierungsmittel für das erfindungsgemäße Verfahren verwendet.

Erfindungsgemäß wird unter dem Begriff "unterschiedliche Wirkstoffe" oder "Wirkstoffe, die voneinander verschieden sind", verstanden, dass jedes in den Hüllliposomen eingeschlossene Liposom mindestens einen Wirkstoff enthält, der von den Wirkstoffen in den anderen Liposomen verschieden ist. Enthalten die eingeschlossenen Liposomen mehrere Wirkstoffe, können somit einzelne Wirkstoffe gleichzeitig auch in mehreren Liposomen enthalten sein. Erfindungsgemäß unterscheidet sich immer die Summe aller Wirkstoffe eines eingeschlossenen Liposoms von der Summe aller Wirkstoffe jedes weiteren eingeschlossenen Liposoms.

Die in den größeren Hüllliposomen eingeschlossenen Liposomen haben naturgemäß eine kleinere Partikelgröße als die umschließenden Hüllliposomen selbst. Erfindungsgemäß können die eingeschlossenen Liposomen verschiedene Größen haben, sowohl die Liposomen mit den unterschiedlichen Wirkstoffen untereinander, als auch die Liposomen einer Gruppe, die denselben Wirkstoff enthalten.

Die eingeschlossenen Liposomen enthalten als Wirkstoffe vorzugsweise pharmazeutische Wirkstoffe, kosmetische Wirkstoffe oder Gemische davon.

Zu den pharmazeutischen Wirkstoffen gehören u. a. dermatologische Wirkstoffe wie zum Beispiel Virustatika oder viruzide Arzneistoffe, Antimykotika, Heparine (z.B. Heparin-Calcium, Heparin-Natrium, niedermolekulare Heparine), Antibiotika, Corticoide, Antiinfektiosa, Aknewirkstoffe, Lokalanästhetika, Antiphlogistika, Antihistaminika oder Antipsoriatika;
systemische Wirkstoffe, wie zum Beispiel nichtsteroidale Analgetika/Antirheumatika (z.B. Diclofenac-Natrium, Diclofenac-Diethylaminsalz, Etofenamat, Flufenaminsäure, 2-Hydroxyethylsalicylat, Ibuprofen, Indomethacin, Piroxicam), Opiatrezeptor-Agonisten und -Antagonisten (z.B. Buprenorphin, Fentanyl, Pentazocin, Pethidin, Tilidin, Tramadol, Naloxon), Histaminantagonisten (z.B. Bamipinlactat, Chlorphenoxamin-HCI, Clemastinhydrogenfumarat, Dimetindenmaleat, Pheniraminhydrogenmaleat), Insuline, regulatorische Peptide und ihre Hemmstoffe (z.B. Hypophysenvorderlappenhormone und ihre Hemmstoffe, Hypophyenhinterlappenhormone, Hypothalamushormone), Sedativa/ Hypnotika (z.B. Diazepam);
oder Wirkstoffe der Gruppe Cytostatika, Cancerostatika, Immunmodulatoren und Vakzine.

Ein bevorzugter dermatologischer Wirkstoff ist beispielsweise Rosmarinsäure oder ein anderer in Pflanzen vorkommender viruzider oder virustatischer Wirkstoff. Ein bevorzugter systemischer Wirkstoff ist beispielsweise ein niedermolekulares oder hochmolekulares Heparin, ein Oligopeptid oder ein Polypeptid. Weitere bevorzugte Wirkstoffe sind Vitamine (E, A, B, C), Muramylpeptide, Doxorubicin, Gentamycin, Gramycidin, Dexamethason, Hydrocortison, Progesteron, Prednisolon bzw. davon abgeleitete Derivate und/oder Säure- bzw. Basenadditionssalze.

Erfindungsgemäß werden als pharmazeutische Wirkstoffe vorzugsweise Rosmarinsäure, Heparin, Oligopeptide, Antibiotika, Aknewirkstoffe, Antihistaminika und Gemische davon verwendet.

Zu den kosmetischen Wirkstoffen gehören z.B. Selbstbräunungsmittel, Radikalfänger, Feuchthaltemittel, Vitamine, Enzyme, pflanzliche Wirkstoffe, Peptide wie Human Oligopeptid-20, L-Dermaxyl® (C12-15 Alkylbenzoate and Tribehenin and Ceramid 2 and PEG-10 Rapeseed Sterol and Palmitoyl Oligopeptide, beispielsweise erhältlich bei Sederma GmbH), Calmosensine® (beispielsweise erhältlich bei Serderma GmbH), insbesondere Peptidgemische wie Adasomes® (Extract & Lecithin & Peptide Mixture (vorgeschlagener INCI-Name), umfassend Pflanzenzellenzytoplasmaextrakte mit einem Anteil an O-6-Methylguanin-DNA-alkyltransferase, beispielsweise erhältlich bei Barnet Products Corporation), Ubichinon Q10, Kreatin, Kreatinin, Camitin, Biotin, Isoflavone, Cardiolipin, Liponsäure, Anti Freezing Proteine, Arctiin, Hopfen- und Hopfen-Malz-Extrakte, verschiedene weitere Pflanzenextrakte wie solche von Orangen, Melonen, Granatapfel, Sojabohnen, Arabidopsis thaliana (beispielsweise erhältlich bei Barnet Products Corporation unter dem Namen Roxisomes), Coffea arabica, Angelica archangelica, Pongamia pinnata, Borago officinalis, Centella asiatica, Peucedanum ostruthium, Calendula officinalis, Inca inchi, Centaurea cyanus, Hedera helix, Thymus vulgaris, Ginkgo biloba, Citrus grandis, Camellia sinensis (Grüntee-Extrakte, insbesondere Camellia Sinensis Leaf Extract, Camellia Sinensis Flower Extract), Maxillaria tenuifolia, Encyalica odoratissima, Nigritella nigra, Gymnadenia austriaca, Plukenetia volubilis, Cynara scolymus, Eucalyptus globulus, Bambusa arundinacea, Papaver rhoeas, Papaver somniferum, Mentha piperita, Mentha aquatica, Passiflora incarnata, Myrtus communis, Quebracho blanco; Vitamin A, Vitamin E, Vitamin C und Vitaminderivate, Eukalyptusöl, Harnstoff und Mineralsalze, insbesondere NaCl, Meeresmineralien sowie Osmolyte, Hyaluronsäure, Pearl Extrakt (Pearl Extract), Plankton Extrakt (Plankton Extract) (Photosomes®, beispielsweise erhältlich bei Barnet Products Corporation), Micrococcus Lysat (Micrococcus Lysate) (Ultrasomes®, beispielsweise erhältlich bei Applied Genetics Inc. Dermatics), Seidenraupenextrakt (Silk Worm Extract), Sericoside, verschiedene Extrakte des Süßholzes, Licochalcone, insbesondere Licochalcone A, Silymarin, Silyphos, Dexpanthenol, Tyrosinsulfat, 8-Hexadecen-1,16-dicarbonsäure, Liponsäure, Liponamid, Kojisäure, Hydrochinon, Arbutin, Fruchtsäuren, insbesondere α-Hydroxy-Säuren (AHAs), Aminoguanidin, Pyridoxamin, Lipofuscine, Nukleinsäure, Oligonukleotide, Purine, Pyrimidine, Dihydroxyaceton, Erythrulose, NO-freisetzende Substanzen, Phytoen, Phytofluen, Neurosporin, Lycopin, β-Carotin, Squalen, Variabilin, Phytansäure und/oder Phytol.

Besonders bevorzugt sind Vitamine, Vitaminderivate, vorzugsweise Vitaminderivate von Vitamin A, C und E, wie z.B. Tocopherylacetat, Tocopherylpalmitat, Ascorbylacetat, Ascorbylpalmitat, Retinylpalmitat, Retinylacetat, Pflanzenextrakte wie Calendula officinalis, Opuntia ficus indica, Punica granatum, Coffea arabica, Angelica archangelica, Pongamia pinnata, Camellia sinensis, Quebracho blanco, Arabidopsis thaliana sowie Photosomes®, Ultrasomes®IIIB, Roxisomes, Adasomes®, Isoflavone, Peptide, Oligopeptide und Enzyme.

Die kosmetischen Wirkstoffe in den erfindungsgemäßen eingeschlossenen Liposomen sind insbesondere kosmetischen Wirkstoffe, wie Vitamine, Vitaminderivate, vorzugsweise Vitaminderivate von Vitamin A, C und E, Pflanzenextrakte, vorzugsweise Arabidopsis thaliana Extrakt, Flavone, Flavonoide, vorzugsweise Isoflavone, Peptide, Enzyme, Plankton Extrakt, Micrococcus Lysat, β-Carotin und Gemische davon.

Bevorzugt enthalten die erfindungsgemäßen Liposome, die in den Hüllliposomen eingeschlossen sind, als kosmetischen Wirkstoff Plankton Extract, Micrococcus Lysat, α-Tocopherol und Vitamin A Palmitat, einen Extrakt von Arabidopsis thaliana und/oder einen Pflanzenzellen-Zytoplasmaextrakt und/oder ein Peptidgemisch. Die Liposomen mit den bevorzugten Wirkstoffen werden vorzugsweise auch für die bevorzugten Ausführungsformen des erfindungsgemäßen Verfahrens verwendet.

Besonders bevorzugt umschließen die erfindungsgemäßen Hüllliposomen vier Liposomen, wobei ein erstes Liposom als kosmetischen Wirkstoff Plankton Extrakt, ein zweites Liposom Micrococcus Lysat, α-Tocopherol und Vitamin A Palmitat, ein drittes Liposom einen Extrakt von Arabidopsis thaliana und ein viertes Liposom einen Pflanzenzellen-Zytoplasmaextrakt und/oder ein Peptidgemisch enthält. Weiter bevorzugt sind sowohl die vier eingeschlossenen Liposomen, als auch das Hüllliposom Phospholipidliposomen. In einer besonders bevorzugten Ausführungsform des erfindungsgemäßen Verfahrens werden diese vier Liposomen in das Hüllliposom eingeschlossen.

Die Bildung der Hüllliposomen erfolgt vorzugsweise durch Zugabe eines Liposombildners in Wasser, kann aber auch nach anderen bekannten Verfahren erfolgen. Die als Liposombildner geeigneten Phospholipide sind z.B. Phosphatidylcholin, Phosphatidylglycerol, Lecithin, Beta, Cholesterylhemisuccinat, Gamma-Dipalmitoyl-alpha-lecithin, Sphingomyelin, Phosphatidylserin, Phosphatidsäure, Phosphatidylethanolamin, Lysolecithin, Lysophosphatidylethanolamin, Phosphatidylinositol, Cephalin, Cardiolipin, Cerebrosid, Dicetylphosphat, Di-oleoylphosphatidylcholin, Di-palmitoylphosphatidylcholin, Dipalmitoylphosphatidylglycerol, Di-oleoylphosphatidylglycerol, Palmitoyl-oleoylphosphatidylcholin, Di-stearoyl-phosphat-idylcholin, Stearoyl-palmitoyl-phosphatidylcholin, Di-palmitoyl-phosphatidylethanolamin, Di-stearoyl-phosphatidylethanolamin, Dimyristoyl-phosphatidylserin, Di-oleoyl-phosphatidylchonin, Oleoyl-palmitoyl-phosphatidylcholin, Phosphatidylcholin/ölsäure und ähnliche.

Die Liposombildner für sowohl die kleineren eingeschlossenen Liposomen, als auch für die größeren Hüllliposomen können gleich oder verschieden sein. Vorzugsweise sind es gleiche Liposombildner. In einer bevorzugten Ausführungsform der Erfindung weisen die Liposomen und/oder die Hüllliposomen als Liposomenbildner die folgenden Phospholipide auf: Phosphatidylcholin, Lecithin, Beta, Sphingomyelin, Lysolecithin und Gemische davon. Speziell bevorzugt als Liposomenbildner für die Liposomen und/oder die Hüllliposomen ist Lecithin.

Zu erfindungsgemäß geeigneten Gelbildnern gehören Carbomer, (auch bezeichnet als polymere Acrylsäure vernetzt mit Allyl-Saccharose, INCI-Name: Polyacrylic Acid) Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit und Gemische davon. Besonders bevorzugt sind Carbomer, das unter sehr vielen Handelsbezeichnungen verfügbar ist, wie z.B. Carbopol®, Pemulen®, Acrysol®, sowie Xanthangummi oder Gemische davon. Besonders bevorzugt wird als Gelbildner Carbomer verwendet.

Als Neutralisierungsmittel wird vorteilhaft Triethanolamin (TEA) eingesetzt.

Ein weiterer Gegenstand der Erfindung ist die kosmetische Basiszusammensetzung, die durch das erfindungsgemäße Verfahren erhältlich ist.

In einer bevorzugten Ausgestaltung der erfindungsgemäßen Liposomen liegt die Partikelgröße der eingeschlossenen Liposomen im Bereich von 60 - 160 nm, insbesondere 80 - 140 nm, besonders bevorzugt 110 - 140 nm. Dabei kann die Partikelgröße für jedes der wenigstens zwei und vorzugsweise vier Liposomen mit darin eingeschlossenen Wirkstoffen unterschiedlich sein, so dass Wirkstoffliposomen mit Partikelgrößen beispielsweise im Bereich von 60 - 110 nm zusammen mit Wirkstoffliposomen mit Partikelgrößen beispielsweise im Bereich von 110 bis 160 nm vorliegen können.

Die größeren Hüllliposomen, die die anderen kleineren Liposomen einschließen, haben eine Partikelgröße, die vorzugsweise im Bereich von 350 - 550 nm liegt, insbesondere im Bereich von 450 - 550 nm.

Unter "einschließen" wird erfindungsgemäß verstanden, dass der größte Teil der kleineren Liposomen, d.h. ca. 80 - 95 %, vorzugsweise 90-95 %, von den größeren Hüllliposomen umschlossen wird, und der Rest der kleineren Liposomen frei in dem nachfolgend gebildeten Gel verbleibt.

Das wässrige Gel, in dem die Liposomen verteilt vorliegen, hat vorzugsweise eine Viskosität von 8.000 - 18.000 mPa·s, insbesondere 10.000 - 18.000 mPa·s. Die Messung der Viskosität erfolgt mit einem Brookfield-Viskosimeter DV-II+ mit den entsprechenden Spindeln TC/TD/TE/TT bei 25°C und im Bereich von 50-75% der Spindelgeschwindigkeiten.

In einer bevorzugten Ausgestaltung der Erfindung sind in den Hüllliposomen drei oder vier Liposomen mit unterschiedlichen Wirkstoffen eingeschlossen.

Die Konzentration der Liposomen liegt insgesamt unter 10 Gew.-%, insbesondere im Bereich von 1 bis 10 Gew.-% bezogen auf das Gesamtgewicht der kosmetischen Basiszusammensetzung.

Vorzugsweise liegt die Konzentration bei 1 bis 8 Gew.-%. Dabei beträgt die Konzentration einer einzelnen Liposomart, also z.B. des ersten Liposoms, nicht mehr als 4 Gew.-%, vorzugsweise 0,5 - 3,5 Gew.-%, insbesondere 0,8 - 2,6 Gew.%. Um eine gute Wirksamkeit zu erreichen, liegen die Konzentrationen von einzelnen Liposomarten üblicherweise beim Stand der Technik deutlich höher, z.B. bei 5 - 10 Gew.-%. Erfindungsgemäß wird für die wenigstens gleiche Wirksamkeit mit einer Menge von weniger als 5 Gew.-% vorteilhafterweise deutlich weniger an Wirkstoff eingesetzt.

Wenn der Gehalt an kleinen Liposomen insgesamt 10 Gew.-% übersteigt, kann keine ausreichend stabile kosmetische Basiszusammensetzung erhalten werden. Wenn die Konzentration des einzelnen kleinen Liposoms unter 0,5 Gew.-% liegt, wird keine ausreichende Wirksamkeit der kosmetischen Basiszusammensetzung erreicht.

Eine besonders bevorzugte Ausführungsform der Erfindung besteht darin, dass die kosmetische Basiszusammensetzung eine kosmetische Basiszusammensetzung ist, worin alle Liposomen Phospolipidliposome sind, ein erstes Liposom einen Extrakt von Arabidopsis thaliana (Roxisomes) umfasst, ein weiteres Liposom Plankton & Lecithin (Photosomes®) umfasst, ein weiteres Liposom ein Peptidgemisch (Adasomes®) umfasst und ein weiteres Liposom Micrococcus Lysate & Lecithin & α-Tocopherol & Vitamin A Palmitate & 2-Phenoxyethanol (Ultrasomes®IIIB) umfasst, und diese vier Liposomen von einem größeren Phospholipidliposom umgeben sind, und dieses größere Phospolipidliposom in einem Gel verteilt vorliegt.

Der genannte Planktonextrakt wird z.B. aus Cyanobakterien der Gattung Anacystes nidulans gewonnen und enthält eine wirksame Menge des Enzyms Photolyase. Dieses Enzym trägt dazu bei, dass infolge von UV-Einstrahlung entstandene Cyclobutanpyrimidin-Dimere unter Einbeziehung von Tageslicht wieder aufgespalten werden und somit ein Reparatureffekt für die Haut erreicht wird.

Die erfindungsgemäß hergestellten kosmetischen Basiszusammensetzungen können zu pharmazeutischen oder kosmetischen Zubereitungen wie Lotionen, Gele, Cremes und andere Emulsionen verarbeitet werden. Die Erfindung betrifft also auch kosmetische Zubereitungen, die die erfindungsgemäße kosmetische Basiszusammensetzung umfassen. Bei der Herstellung der kosmetischen Zubereitung sind Temperaturen über 40°C und Rührgeschwindigkeiten von mehr als 500 U/min zu vermeiden. Die erfindungsgemäßen kosmetischen Zubereitungen werden durch Verarbeitung der erfindungsgemäßen kosmetischen Basiszusammensetzung mit üblichen Hilfsstoffen, Trägerstoffen oder deren Gemischen, wie sie üblicherweise in dermatologischen oder kosmetischen Zubereitungen eingesetzt werden, hergestellt. Dazu gehören beispielsweise Wasser, Konservierungsmittel, Farbstoffe, Pigmente mit färbender Wirkung, Verdickungsmittel, Erweichungsmittel, Duftstoffe, Alkohole, Polyole, Ester, Elektrolyte, Gelbildner, polare und unpolare Öle, Polymere, Copolymere, Emulgatoren, Wachse, Stabilisatoren, Füllstoffe, Lichtschutzmittel.

Zu den Füllstoffen gehören beispielsweise Talkum, Glimmer, Kieselsäure, Teflon, Stärke, Perlmutt, Nylonpulver, Polyethylenpulver, Bornitrid, Mikroperlen, Mikroschwämme, Mikrokugeln aus Silikonharz, Mikroperlen aus Kieselsäure, gemahlene Pflanzenfasern und ähnliche.

Die für die Zubereitungen eingesetzten Öle können übliche kosmetische Öle sein, wie z.B. ein Mineralöl, hydriertes Polyisobuten, synthetisches oder aus Naturprodukten hergestelltes Squalan, kosmetische Ester oder Ether, die verzweigt oder unverzweigt, gesättigt oder ungesättigt sein können, pflanzliche Öle, oder Gemische zweier oder mehrerer davon.

Besonders geeignete Öle sind beispielsweise Siliconöle, Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether sowie pflanzliche Öle, wie Calendulaöl, Jojobaöl, Avocadoöl, Macadamianussöl, Rizinusöl, Kakaobutter, Kokosnussoil, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinussöl, Distelöl und Gemische davon.

Je nachdem, welche Öle ausgewählt werden, werden die kosmetischen Eigenschaften der festen Zusammensetzung beeinflusst, wie Transparenzgrad, Weichheit, Härte, Spreitungswirkung.

Besonders bevorzugt sind Siliconöle wie Dimethicone, mit Polyethylenglycol modifiziertes Dimethicone, Cyclomethicone (alle beispielsweise erhältlich bei DowCorning) und Gemische davon.

Als Erweichungsmittel können normalerweise eine Vielzahl von Verbindungen eingesetzt werden, wie beispielsweise Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie z.B. Maisöl, Baumwollsamenöl, Olivenöl, mineralische Öle, Butylmyristat, Palmitinsäure usw..

Die Zubereitung kann auch übliche Feuchthaltemittel enthalten, wie beispielsweise Glycerin, Butylenglycol, Propylenglycol, Dipropylenglycol und Gemische davon, sowie Hyaluronsäure, Lactose, Glycin, Allantoin, Sorbitol, Polyethylenglycole wie z.B. PEG-8. Besonders bevorzugt sind auch Pflanzenextraktgemische, wie z.B. solche aus Punica granatum, Evernia furfuracea und Evernia prunastri.

Für die Herstellung eines Gels können auch die oben bereits genannten Gelbildner verwendet werden, vorzugsweise Carbomer und Xanthangummi.

Wachse können für die Herstellung einer Zubereitung verwendet werden. Die Wachse werden ausgewählt unter natürlichen pflanzlichen Wachsen, tierischen Wachsen, natürlichen und synthetische Mineralwachsen und synthetischen Wachsen. Dazu gehören beispielsweise Carnaubawachs, Candellilawachs, Ozokerit, Bienenwachs, Montanwachs, Wollwachs, Ceresin, Mikrowachse, Hartparaffin, Petrolatum, Siliconwachs, Polyethylenglycol- oder -glycolesterwachse. Besonders bevorzugt sind Siliconwachse, mikrokristalliner Wachs und Bienenwachs.

Pigmente, Pigmentgemische oder Pulver mit pigmentartiger Wirkung, worunter auch solche mit Perlglanz-Effekt zu verstehen sind, können in den Zubereitungen mit der erfindungsgemäßen kosmetischen Basiszusammensetzung ebenfalls eingesetzt werden. Diese Pigmente umfassen z.B. Eisenoxide, natürliche Aluminiumsilicate; Calcium-Aluminium-Borsilicate und deren Gemische mit TiO₂, SiO₂ und SnO₂; Titandioxid, Glimmer, Kaolin, manganhaltige Tone, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver; pulverförmige natürliche organische Verbindungen wie z.B. gemahlene Festalgen, gemahlene Pflanzenteile, verkapselte und unverkapselte Getreidestärken.

Die Zubereitungen mit der erfindungsgemäßen kosmetischen Basiszusammensetzung können weiterhin Antioxidationsmittel und/oder Radikalfänger enthalten. Dazu gehören beispielsweise Vitamine wie Vitamin C und Derivate davon, z.B. Ascorbylacetat, - phosphat und -palmitat, Magnesiumascorbylphosphat; Vitamin A und Derivate davon; Folsäure und deren Derivate; Vitamin E und deren Derivate, wie z.B. Tocopherylacetat; Flavone oder Flavonoide; Aminosäuren, wie z.B. Histidin, Glycin, Tyrosin, Tryptophan und Derivate davon; Imidazole wie z.B. cis- oder trans-Urocaninsäure und deren Derivate; Peptide wie z.B. D,L-Carnosin, D-Carnosin, L-Carnosin sowie Oligopeptide mit 3-20 Aminosäuren und deren Derivate; Carotinoide und Carotine, wie z.B α-Carotin, β -Carotin, Lycopin; Harnsäure und Derivate davon; α-Hydroxysäuren wie z.B. Citronensäure, Milchsäure, Apfelsäure; α-Hydroxyfettsäuren wie z.B. Palmitinsäure, Phytinsäure, Lactoferrin; Stilbene und deren Derivate; Mannose und deren Derivate; Liponsäure und deren Derivate, z.B. Dihydroliponsäure; Ferulasäure und deren Derivate; Thiole wie z.B. Glutathion, Cystein, Cystin und deren Ester sowie viele Pflanzenextrakte.

Ein besonders geeigneter Radikalfänger ist eine Wirkstoffzubereitung mit einem Gehalt an einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden und Wasser.

Ebenfalls bevorzugt ist eine Wirkstoffzubereitung mit Pflanzenextrakten, die ein liposomfreies Gemisch von Pflanzenextrakten auf alkoholischer Basis darstellt, bestehend aus dem Extrakt grüner Kaffeebohnen (Coffea Arabica Seed Extract, Coffea Arabica Leaf/Seed Extract), dem Extrakt von Blättern von Camellia sinensis, dem Extrakt von Pongamia pinnata und dem Extrakt der Wurzeln von Angelica archangelica und Alkohol im Verhältnis von 0,2 : 0,2 : 0,2 : 0,2 : 99,2, wobei der Alkohol ein niederer einwertiger Alkohol wie Ethanol ist.

Ein weiteres bevorzugtes Antioxidationsmittel ist ein in Lecithin verkapseltes Gemisch aus (in Gew.-%) 2 % Angelica archangelica-Wurzelextrakt (INCI: Angelica archangelica root extract, CAS number 84775-41-7), 2 % Pongamia pinnata-Samenextrakt (INCI: Pongamia pinnata seed extract), 2 % Camelia sinensis-Blattextrakt (INCI: Camelia sinensis Leaf Extract, CAS number 84650-60-2), 2 % Coffea arabica-Samenextrakt (INCI: Coffea arabica (coffee) seed extract, CAS number 84650-00-0), wobei außerdem 8 % Glycerin, 8,25 % Alkohol denaturiert, Antioxidationsmittel und Hilfsstoffe enthalten sind.

Die Zubereitungen mit den erfindungsgemäßen kosmetischen Basiszusammensetzungen können vorteilhaft auch wasser- und/oder öllösliche Lichtschutzfilter in Form von UVA- oder UVB-Filtern oder Gemischen davon enthalten. Zu vorteilhaften öllöslichen UVB-Filtern gehören 4-Aminobenzoesäure-Derivate wie z.B. der 4-(Dimethylamino)-benzoesäure-(2-ethylhexyl) ester; Ester der Zimtsäure wie z.B. der 4-Methoxyzimtsäure(2-ethylhexyl)ester; Benzophenon-Derivate wie z.B. 2-Hydroxy-4-methoxybenzophenon; 3-Benzylidencampher-Derivate wie z.B. 3-Benzylidencampher.

Bevorzugte öllösliche UV Filter sind Benzophenon-3, Butyl-Methoxybenzoylmethan, Octyl Methoxycinnamat, Octyl Salicylat, 4-Methylbenzyliden Campher, Homosalat und Octyl Dimethyl PABA.

Wasserlösliche UVB-Filter sind z.B. Sulfonsäurederivate von Benzophenon oder von 3-Benzylidencampher oder Salze wie beispielsweise das Na- oder K-Salz der 2-Phenylbenzimidazol-5-sulfonsäure.

Zu UVA-Filtern gehören Dibenzoylmethan-Derivate wie z.B. 1-Phenyl-4-(4'-isopropylphenyl)propan-1,3-dion, Butyl Methoxybenzoyl-methan oder Menthyl Anthranilat.

Besonders bevorzugt sind Benzophenon-3, Butyl Methoxydibenzoylmethan, Octyl Methoxycinnamat, Octyl Salicylat, 4-Methylbenzyliden Campher, Homosalat, Octocrylen, Ethylhexyl Methoxycinnamat, Isoamyl-p-Methoxycinnamat, Octyl Dimethyl PABA, Ethylhexyltriazon, Diethylhexyl Butamido Triazon, Ethylhexyl Salicylat, Methylen Bis-Benzotriazolyl Tetramethylbutylphenol, Disodium Phenyl Dibenzimidazol Tetrasulfonat, Bis-Ethylhexyloxyphenol Methoxyphenyl Triazin.

Weiterhin können auch Breitbandfilter eingesetzt werden, wie z.B. Bis-Resorcinyltriazin-Derivate, oder auch Benzoxazole.

Weiterhin einsetzbar als Lichtschutzfilter sind anorganische Pigmente auf Basis von Metalloxiden, wie TiO₂, SiO₂, ZnO, Fe₂O₃, ZrO₂, MnO, Al₂O₃, die auch im Gemisch verwendet werden können.

Der Anteil der kosmetischen Basiszusammensetzung in einer erfindungsgemäßen kosmetischen Zubereitung liegt allgemein im Bereich von 1 bis 20 Gew.-%, bezogen auf das Gesamtgewicht der Zubereitung, vorzugsweise im Bereich von 2 bis 12 Gew.-%, insbesondere im Bereich von 2 bis 6 Gew.-%.

Die Zubereitungen können in Form einer Milch, einer Lotion, einer Creme und sonstiger üblicher Zubereitungen im genannten Viskositätsbereich liegen.

Ein weiterer Gegenstand der Erfindung ist die Verwendung der kosmetischen Basiszusammensetzung der Erfindung für kosmetische Zubereitungen zur Verbesserung der Hautregenerierung und/oder als Anti-Alterungsmittel.

Weiterhin kann die erfindungsgemäße kosmetische Basiszusammensetzung auch als Hilfsstoff in pharmazeutischen Zubereitungen verwendetet werden. Besonders bevorzugt ist die Verwendung der kosmetischen Basiszusammensetzung, wenn die Haut durch den pharmazeutischen Wirkstoff stark beansprucht wird. Der Anteil der kosmetischen Basiszusammensetzung in einer pharmazeutischen Zubereitung liegt allgemein im Bereich von 1 bis 20 Gew.-%, bezogen auf das Gesamtgewicht der Zubereitung, vorzugsweise im Bereich von 2 bis 12 Gew.-%, insbesondere im Bereich von 2 bis 6 Gew.-%.

Die Erfindung soll nachstehend durch Beispiele näher erläutert werden. Alle Angaben erfolgen in Gewichtsprozent, sofern nichts anderes angegeben ist.

### Beispiel 1

In einen Rührbehälter, der mit einem Planetenrührer und einem Kühlmantel ausgestattet ist, wird deionisiertes Wasser gegeben. Die Temperatur wird bei 25°C gehalten und dabei werden nacheinander unter Rühren mit 120 U/min eingetragen:
(1) 4 Gew.-% Liposomflüssigkeit mit Photosomes® (INCI: Plankton Extract & Lecithin),
(2) 4 Gew.-% Liposomflüssigkeit mit Ultrasomes® IIIB (INCI: Micrococcus Lysate & Lecithin & α-Tocopherol & Vitamin A Palmitate & 2-Phenoxyethanol),
(3) 1 Gew.-% Liposomflüssigkeit mit Roxisomes® (INCI: Arabidopsis Extract & Lecithin & Water),
(4) 1 Gew.-% Liposomflüssigkeit mit Adasomes® (INCI: Extract & Lecithin & Peptide Mixture (vorgeschlagener INCI-Name).

Die Rührgeschwindigkeit wird auf 75 U/min verringert, und es werden 0,9 1 Lecithin mit einer Geschwindigkeit von etwa 2,8 l/h zugegeben, wobei beim Rühren darauf geachtet wird, dass keine Verwirbelungen des gerührten Gemisches auftreten.

Nach Beendigung der Lecithinzugabe wird bei etwa gleicher Rührgeschwindigkeit 0,2 Gew.-% Carbomer über einen Zeitraum von 40 min zugegeben und bis zum Erreichen eines homogenen Gemisches weitergerührt. Die Homogenität lässt sich lichtmikroskopisch bei einer Probennahme erkennen.

Im Anschluss daran werden 0,15 - 0,2 Gew.-% Triethanolamin als Neutralisierungsmittel zugegeben, und die Rührgeschwindigkeit wird für 40 min auf 720 U/min erhöht. Die Temperatur wird dabei bei etwa 35°C gehalten.

Man erhält die kosmetische Basiszusammensetzung mit einer Brookfield-Viskosität von 16.200 mPa·s, gemessen bei 25°C mit einer Spindel TT (Nr. 6, Geschwindigkeit 10).

### Beispiel 2

Es wurde wie im Beispiel 1 gearbeitet mit folgenden Konzentrationen: (1) 2 Gew.-%; (2) 2 Gew.-%, (3) 2 Gew.-%; (4) 2 Gew.-%.

### Beispiel 3

Es wurde wie im Beispiel 1 gearbeitet mit folgenden Konzentrationen: (1) 2 Gew.-%; (2) 2 Gew.-%, (3) 4 Gew.-%; (4) 2,5 Gew.-%.

### Beispiel 4

Es wurde wie im Beispiel 1 gearbeitet mit folgenden Konzentrationen: (1) 2 Gew.-%; (2) 2 Gew.-%, (3) 4 Gew.-%; (4) 2 Gew.-%.

### Beispiel 5

Es wurde nach der Verfahrensweise von Beispiel 1 gearbeitet. Die Liposomflüssigkeiten, für die als Liposombildner Phosphatidylcholin/Lysolecithin eingesetzt wurde, enthielten als Wirkstoffe (1) 1 Gew.-% Gentamycin, (2) 2,5 Gew.-% Retinol und (3) 1,5 Gew.-% Rosmarinsäure.

### Beispiel 6

Es wurde nach der Verfahrensweise von Beispiel 1 gearbeitet. Die Liposomflüssigkeiten, für die als Liposombildner Lecithin eingesetzt wurde, enthielten als Wirkstoffe (1) 1,5 Gew.-% Isoflavone 150 (Lucas Meyer), (2) 0,8 Gew.-% Heparin-Na, (3) 3,5 Gew.-% Vitamin C und (4) 1,5 Gew.-% Aloe Parryi Extrakt.

### Beispiel 7

Es wurde nach der Verfahrensweise von Beispiel 1 gearbeitet. Die Liposomflüssigkeiten, für die als Liposombildner Lecithin eingesetzt wurde, enthielten als Wirkstoffe (1) 1 Gew.-% Retinol; (2) 2 Gew.-% Tocopherylacetat; (3) 1,5 Gew.-% 2-Dermaxyl®; (4) 0,8 Gew.-% Isoflavon.

### Beispiel 8 Kosmetische Zubereitung

| | |
|---|---|
| Wasser | q.s. ad 100 |
| PEG-12 Dimethicone | 1,2 |
| Bis-PEG-18-Methyl Ether Dimethyl Silane BHT (Wax) | 2,4 |
| PEG-8 | 6,3 |
| Trisodium EDTA | 0,1 |
| Butylene Glycol | 5,0 |
| Carbomer | 0,25 |
| Xanthan Gum | 1,1 |
| Glycerin | 1,8 |
| Ethanol | 3,5 |
| PEG-40 Hydrogenated Castor Oil | 0,3 |
| 1,2-Hexanediol & Caprylyl Glycol | 0,6 |
| Punica Granatum Extract & Evernia Furfuracea Extract & Evernia Prunastri Extract & Perfumes | 0,8 |
| Kosmetische Basiszusammensetzung nach Beispiel 1 | 3,0 |
| Potassium PCA & Opuntia Ficus India Stem Extract & Water | 1,8 |
| Human Oligopeptide-20 (Pepha-Timp®) | 0,15 |
| RPF-Komplex* | 0,7 |
| Calciumaluminium Borosilicate | 0,3 |
| Triethanolamin | 0,25 |

| | |
|---|---|
| * bestehend aus einer Wirkstoffzubereitung mit hohem Radikalschutzfaktor mit einem Gehalt an einem durch Extraktion der Rinde von Quebracho blanco und nachfolgender enzymatischer Hydrolyse gewonnenem Produkt, das wenigstens 90 Gew.-% Proanthocyanidin-Oligomere und höchstens 10 Gew.-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser gemäß dem Herstellungsbeispiel 1 des Wirkstofifkomplexes in der WO99/66881. | |

Die einzelnen Bestandteile der Zubereitung wurden nacheinander bei etwa 40°C verrührt, wobei die Rührgeschwindigkeit bei 200 U/min lag und die Temperatur 38 - 40°C betrug. Nach dem Homogenisieren mit etwa 1.500 U/min wurde Triethanolamin zugegeben und nochmals gerührt. Zum Schluss erfolgte die Zugabe des RPF-Komplexes und der kosmetischen Basiszusammensetzung bei etwa 30°C unter Rühren mit 300 - 400 U/min mit einem Planetenrührer bis zum Erreichen der Homogenität (ca. 1 - 3 Minuten).

### Beispiel 9 Kosmetische Creme

| | |
|---|---|
| Wasser | q.s. ad 100 |
| EDTA | 0,1 |
| Glycerin | 5,0 |
| Sodium Hyaluronate | 0,15 |
| Sodium Polyacrylate | 1,5 |
| Beheneth-25 | 4,0 |
| Cetearyl Alcohol | 1,5 |
| Hydroxyoctacosanyl Hydroxystearate | 3,5 |
| Petrolatum | 3,0 |
| Dimethicone | 3,0 |
| Optiphen plus (Phenoxyethanol & Caprylyl Glycol & Sorbic Acid) | 1,5 |
| Cyclohexasiloxane & Cyclopentasiloxane & Cyclotetrasiloxane | 2,0 |
| RPF-Komplex* | 1,5 |
| Kosmetische Basiszusammensetzung nach Beispiel 3 | 5,0 |

| | |
|---|---|
| * siehe Beispiel 8 | |

Die einzelnen Bestandteile wurden in der Reihenfolge der Auflistung unter Rühren bei 200 U/min zugegeben. Bei 30°C wurden mit 300-400 U/min eines Planetenrührers der RPF-Komplex und die kosmetische Basiszusammensetzung zugegeben.

### Beispiel 10 Kosmetische Creme II

| | |
|---|---|
| Wasser | q.s. ad 100 |
| EDTA | 0,1 |
| Glycerin | 5,0 |
| Sodium Hyaluronate | 0,15 |
| Sodium Polyacrylate | 1,5 |
| Beheneth-25 | 4,0 |
| Cetearyl Alcohol | 1,5 |
| Hydroxyoctacosanyl Hydroxystearate | 3,5 |
| Petrolatum | 3,0 |
| Dimethicone | 3,0 |
| Optiphen plus (Phenoxyethanol & Caprylyl Glycol & Sorbic Acid) | 1,5 |
| Cyclohexasiloxane & Cyclopentasiloxane & Cyclotetrasiloxane | 2,0 |
| RPF-Komplex* | 1,5 |
| Kosmetische Basiszusammensetzung nach Beispiel 4 | 5,0 |

| | |
|---|---|
| * siehe Beispiel 8 | |

Die einzelnen Bestandteile wurden in der Reihenfolge der Auflistung unter Rühren bei 200 U/min zugegeben. Bei 30°C wurden mit 300-400 U/min eines Planetenrührers der RPF-Komplex und die kosmetische Basiszusammensetzung zugegeben.

### Beispiel 11 Vergleichsbeispiel I

Es wurde eine kosmetische Zubereitung gemäß Beispiel 8 hergestellt (Probe A). Es wurde eine weitere kosmetische Zubereitung ähnlich wie in Beispiel 8 hergestellt, bei der jedoch anstelle der kosmetischen Basiszusammensetzung nach Beispiel 1 die gleichen Mengen an einzelnen Liposomen eingesetzt wurden (Probe B).

Es wurde ein Anwendertest mit 40 Teilnehmerinnen im Alter von 44 bis 58 Jahren durchgeführt. 20 Teilnehmerinnen (Gruppe A) wandten die Zubereitung Probe A im Gesicht an, insbesondere rund um die Augenpartie. Die andere Gruppe (B) wandte die Zubereitung Probe B an den gleichen Flächen an.

Die Zubereitungen wurden zweimal täglich morgens und abends mit einer durchschnittlichen Menge von 2-4 mg/cm² aufgetragen. Die Messung nach 12 Stunden wurde nach einmaliger Anwendung durchgeführt. Es erfolgte eine computerisierte Auswertung über digitalisierte Aufnahmen von den Augenwinkeln der Probandinnen und einer weiteren ausgewählten Gesichtspartie. Die Faltenreduzierung beider Flächen wurde zusammengefasst in Prozent festgestellt.

Die Ergebnisse sind in Tabelle 1 dargestellt.

**Tabelle 1**

| | Faltenreduzierung [%] | | |
|---|---|---|---|
| | | | |
| | | Gruppe A | Gruppe B |
| Behandlungsbeginn | | 0 | 0 |
| nach | 12 Stunden | 8 | 3 |
| nach | 3 Tagen | 10-13 | 7-8 |
| | 7 Tagen | 15-19 | 9-12 |
| | 10 Tagen | 24-26 | 14-16 |
| | 20 Tagen | 30-32 | 22-23 |
| | 28 Tagen | 38-42 | 28-30 |
| | 40 Tagen | 32-35 | 18-20 |

Tabelle 1 zeigt deutlich die Überlegenheit der Probe A mit der erfindungsgemäßen kosmetische Basiszusammensetzung gegenüber der Einzelanwendung der vier Liposomen in der Probe B. Die Behandlung mit der Probe B führt zu einem erwarteten Anstieg der Faltenreduzierung im Zeitraum bis zu einem Monat von etwa 30 %. Dagegen zeigt die erfindungsgemäße Probe A bereits in den ersten Stunden/Tagen einen überraschend schnellen Anstieg, der nach 28 Tagen bis auf über 40 % führt. Dieses signifikante und überraschende Ergebnis verdeutlicht das hohe Potential der kosmetischen Basiszusammensetzung, die mit gleichen Wirkstoffmengen schnellere und in der Langzeitwirkung bessere Ergebnisse erzielt. Die Langzeitwirkung ist insbesondere aus den Messwerten nach 40 Tagen ersichtlich, da in der Zeit zwischen 28 und 40 Tagen keine Anwendung der Proben erfolgte.

### Beispiel 12 Vergleichsbeispiel II

Die im Beispiel 1 angegebenen Liposomen in den genannten Mengen werden bei 25 °C nacheinander unter Rühren bei 250 U/min in Wasser eingetragen. Es werden danach 0,9 l Lecithin mit einer Geschwindigkeit von 4 l/h unter Rühren mit 150 U/min eingetragen und anschließend bei gleicher Geschwindigkeit 0,2 Gew.-% Carbomer über einen Zeitraum von 15 min.

Das dabei erhaltene Gel war nicht homogen. Beim Stehenlassen des Gels über 24 Stunden sedimentierten die Liposomen. Daraus war ersichtlich, dass sich keine oder nur unzureichend wenige größere Hüllliposomen gebildet hatten.

## Patentansprüche

1. Verfahren zur Herstellung einer kosmetischen Basiszusammensetzung umfassend Hüllliposomen mit einer Partikelgröße von 250 - 600 nm in einem wässrigen Gel mit einer Viskosität im Bereich von 4.000 bis 20.000 mPa·s, die in ihrem wässrigen Volumen drei oder vier Liposomen einschließen, die jeweils mindestens einen Wirkstoff in ihrem wässrigen Volumen enthalten, wobei die in den eingeschlossenen Liposomen enthaltenen Wirkstoffe voneinander verschieden sind und die eingeschlossenen Liposomen eine Partikelgröße im Bereich von 50 - 200 nm haben, **dadurch gekennzeichnet, dass** die drei oder vier Liposomen unter Rühren in Wasser eingebracht werden und anschließend in das Liposom-Wasser-Gemisch ein Liposombildner, ein Gelbildner und ein Neutralisierungsmittel eingebracht werden, wobei die Liposomen nacheinander unter Rühren mit 100-150 U/min in einem Zeitraum von 10 bis 20 Minuten in das Wasser eingebracht werden; danach der Liposombildner unter Rühren mit 50-90 U/min mit einer Geschwindigkeit von 1,5-3,5 l/h in das Liposom-Wasser-Gemisch eingebracht und die Temperatur während dieser Zeit zwischen 15 und 40 °C gehalten wird; danach der Gelbildner unter Rühren mit 50-90 U/min über einen Zeitraum von 20-50 Minuten bis zum Erreichen eines homogenen Gemisches eingebracht wird; danach unter Rühren bei der gleichen Rührgeschwindigkeit das Neutralisierungsmittel zugegeben wird; und dann die Rührgeschwindigkeit für 30-60 Minuten auf 500-1.000 U/min erhöht und dabei die Temperatur unter 40 °C gehalten wird, wobei als Liposombildner Phospholipide eingesetzt werden.

2. Verfahren nach Anspruch 1, **dadurch gekennzeichnet, dass** das Neutralisierungsmittel eine schwache Base, vorzugsweise Mono-, Di- oder Triethanolamin, Trimethylamin, Triethylamin, Tripropylamin, Tributylamin, Imidazol oder Pyridin, besonders bevorzugt Triethylamin, ist.

3. Verfahren nach einem der Ansprüche 1 oder 2, **dadurch gekennzeichnet, dass** die Wirkstoffe pharmazeutische Wirkstoffe, kosmetische Wirkstoffe oder Gemische davon sind.

4. Verfahren nach Anspruch 3, **dadurch gekennzeichnet, dass** die kosmetischen Wirkstoffe Vitamine, Vitaminderivate, vorzugsweise Vitaminderivate von Vitamin A, C und E, Pflanzenextrakte, vorzugsweise Arabidopsis thaliana Extrakt, Flavone, Flavonoide, vorzugsweise Isoflavone, Peptide, Enzyme, Plankton Extrakt, Micrococcus Lysat, β-Carotin oder Gemische davon sind.

5. Verfahren nach einem der Ansprüche 1 bis 4, **dadurch gekennzeichnet, dass** die Liposomen und/oder die Hüllliposomen als Liposombildner Phosphatidylcholin, Lecithin, Breta, Sphingomyelin, Lysolecithin und Gemische davon aufweisen.

6. Verfahren nach einem der Ansprüche 1 bis 5, **dadurch gekennzeichnet, dass** ein erstes Liposom als kosmetischen Wirkstoff Plankton Extrakt, ein zweites Liposom Micrococcus Lysat, α-Tocopherol und Vitamin A Palmitat, ein drittes Liposom einen Extrakt von Arabidopsis thaliana und ein viertes Liposom einen Pflanzenzellen-Zytoplasmaextrakt und/oder ein Peptidgemisch enthält.

7. Verfahren nach einem der Ansprüche 1 bis 6, **dadurch gekennzeichnet, dass** die Konzentration der in den Hüllliposomen eingeschlossenen Liposomen im Bereich von 1 bis 10 Gew.-%, vorzugsweise im Bereich von 1 bis 8 Gew.-%, bezogen auf das Gesamtgewicht der kosmetischen Basiszusammensetzung liegt.

8. Verfahren nach einem der Ansprüche 1 bis 7, **dadurch gekennzeichnet, dass** der Gelbildner Carbomer, Xanthan Gum oder ein Gemisch davon, vorzugsweise Carbomer ist.

9. Kosmetische Basiszusammensetzung erhältlich nach dem Verfahren gemäß einem der Ansprüche 1 bis 8.

10. Kosmetische Zubereitung, **dadurch gekennzeichnet, dass** sie die kosmetische Basiszusammensetzung nach Anspruch 9 umfasst.

11. Nicht-therapeutische Verwendung der kosmetischen Basiszusammensetzung nach Anspruch 9 oder der kosmetischen Zubereitung nach Anspruch 10 zur Verbesserung der Hautregenerierung und/oder als Anti-Alterungsmittel.

## Claims

1. A method for manufacturing a cosmetic base composition comprising enveloping liposomes having a particle size of 250 - 600 nm in an aqueous gel with a viscosity in the range from 4,000-20,000 mPas, the enveloping liposomes embedding in its aqueous volume three or four liposomes, each containing at least one active ingredient in the aqueous volume thereof, wherein the active ingredients in the embedded liposomes differ from each other and the embedded liposomes have a particle size in the range from 50 - 200 nm, **characterized in that** the three or four liposomes are introduced into water while stirring, then a liposome forming agent, a gelling agent and a neutralizing agent are added to the liposome-water mixture, wherein the liposomes are added to the water one after the other with stirring at 100-150 rpm within a period of 10-20 minutes; then the liposome forming agent is added to the liposome-water mixture at a rate of 1.5-3.5 l/hour while stirring at 50-90 rpm, and the temperature is maintained at 15-40 °C throughout this time; then the gelling agent is added over a period of 20-50 minutes while stirring at 50-90 rpm until a homogeneous mixture is obtained; then the neutralizing agent is added while stirring at the same stirring speed; then the stirring speed is increased to 500-1000 rpm for 30-60 minutes and thereby the temperature is maintained below 40 °C, wherein phospholipids are used as liposome forming agents.

2. The method according to claim 1, **characterized in that** the neutralizing agent is a weak base, preferably mono-, di- or triethanolamine, trimethylamine, triethylamine, tripropylamine, tributylamine, imidazole or pyridine, particularly preferred triethylamine.

3. The method according to either of claims 1 or 2, **characterized in that** the active substances are pharmaceutical active substances, cosmetic active substance or mixtures thereof.

4. The method according to claim 3, **characterized in that** the cosmetic active substances are vitamins, vitamin derivatives, preferably vitamin derivatives of vitamin A, C and E, plant extracts, preferably Arabidopsis thaliana extract, flavones, flavonoids, preferably isoflavones, peptides, enzymes, plankton extract, micrococcus lysate, β-carotene or mixtures thereof.

5. The method according to any one of claims 1 to 4, **characterized in that** the liposomes and/or the enveloping liposomes include phosphatidylcholin, lecithin, beta, sphingomyelin, lysolecithin and mixtures thereof as liposome forming agents.

6. The method according to any one of claims 1 to 5, **characterized in that** a first liposome contains plankton extract as cosmetic active substance, a second liposome contains micrococcus lysate, α-tocopherol and vitamin A palmitate, a third liposome contains an extract of Arabidopsis thaliana and a fourth liposome contains a plant cell cytoplasm extract and/or a peptide mixture.

7. The method according to any one of claims 1 to 6, **characterized in that** the concentration of the liposomes embedded in the enveloping liposomes is in the range from 1 to 10% by weight, preferably in the range from 1 to 8% by weight relative to the total weight of the cosmetic base composition.

8. The method according to any one of claims 1 to 7, **characterized in that** the gelling agent is carbomer, xanthan gum or a mixture thereof, preferably carbomer.

9. Cosmetic base composition obtainable with the method according to any one of claims 1 to 8.

10. Cosmetic preparation, **characterized in that** it contains the cosmetic base composition according to claim 9.

11. Non-therapeutic use of the cosmetic base composition according to claim 9 or the preparation according to claim 10 for improving skin regeneration and/or as an anti-aging substance.

## Revendications

1. Procédé de fabrication d'une composition cosmétique de base comprenant des liposomes formant enveloppe d'une dimension de particules de 250 à 600 nm dans un gel aqueux d'une viscosité de l'ordre de 4.000 à 20.000 mpa, qui dans leur volume aqueux renferment trois ou quatre liposomes qui contiennent chacun au moins un principe actif dans leur volume aqueux, les principes actifs contenus dans les liposomes renfermés étant différents les uns des autres et les liposomes renfermés ayant une dimension de particules de l'ordre de 50 à 200 nm, **caractérisé en ce qu'**on introduit les trois ou quatre liposomes dans de l'eau sous agitation et **en ce que** par la suite, on introduit dans le mélange eau/liposomes un constituant de liposomes, un gélifiant et un agent neutralisant, les liposomes étant introduits successivement dans l'eau, sous agitation à raison de 100 à 150 t/mn, pendant une période de 10 à 20 mn ; **en ce que** par la suite, on introduit les constituants de liposomes dans le mélange eau/liposomes sous agitation à raison de 50 à 90 t/mn, à une vitesse de 1,5 à 3,5 l/h et **en ce que** pendant cette période, on maintient la température entre 15 et 40 °C ; **en ce qu'**on introduit par la suite le gélifiant sous agitation à raison de 50 à 90 t/mn pendant une période de 20 à 50 mn, jusqu'à l'obtention d'un mélange homogène ; **en ce que** par la suite, sous agitation à la même vitesse d'agitation, on ajoute l'agent neutralisant; et **en ce qu'**on augmente ensuite la vitesse d'agitation à de 500 à 1.000 t/mn pendant de 30 à 60 mn, en maintenant à cet effet la température à une valeur inférieure à 40 °C, des phospholipides étant mis en oeuvre en tant que constituants de liposomes.

2. Procédé selon la revendication 1, **caractérisé en ce que** l'agent neutralisant est une base faible, de préférence une monoéthanolamine, une diéthanolamine ou une triéthanolamine, une triméthylamine, une triéthylamine, une tripropylamine, une tributylamine, de l'imidazole ou de la pyridine, de manière particulièrement préférée une triéthylamine.

3. Procédé selon l'une quelconque des revendications 1 ou 2, **caractérisé en ce que** les principes actifs sont des principes actifs pharmaceutiques, des principes actifs cosmétiques ou des mélanges de ces derniers.

4. Procédé selon le revendication 3, **caractérisé en ce que** les principes actifs cosmétiques sont des vitamines, des dérivés vitaminiques, de préférence des dérivés vitaminiques des vitamine A, C et E, des extraits végétaux, de préférence de l'extrait de l'arabette de Thalius, des flavones, des flavonoïdes, de préférence des isoflavones, des peptides, des enzymes, de l'extrait de plancton, de la micrococcus lysate, du β-carotène ou des mélanges de ces derniers.

5. Procédé selon l'une quelconque des revendications 1 à 4, **caractérisé en ce que** les liposomes et/ou les liposomes formant enveloppe comportent en tant que constituants de liposomes de la phosphatidylcholine, de la lécithine, du breta, de la sphingomyéline, de la lysolécithine et des mélanges de ces derniers.

6. Procédé selon l'une quelconque des revendications 1 à 5, **caractérisé en ce qu'**un premier liposome contient en tant que principe actif cosmétique de l'extrait de plancton, un deuxième liposome de la micrococcus lysate, de l'α-tocophérol et du palmitate A, un troisième liposome un extrait de l'arabette de Thalius et un quatrième liposome contient un extrait de cytoplasme de cellules végétales et/ou un mélange de peptides.

7. Procédé selon l'une quelconque des revendications 1 à 6, **caractérisé en ce que** la concentration des liposomes renfermés dans les liposomes formant enveloppe se situe dans l'ordre de 1 à 10 % en poids, de préférence dans l'ordre de 1 à 8 % en poids en rapport au poids total de la composition cosmétique de base.

8. Procédé selon l'une quelconque des revendications 1 à 7, **caractérisé en ce que** le gélifiant est du carbomère, de la gomme de xanthane ou un mélange de ces derniers, de préférence du carbomère.

9. Composition cosmétique de base, pouvant être obtenue d'après le procédé selon l'une quelconque des revendications 1 à 8.

10. Préparation cosmétique, **caractérisée en ce qu'**elle comprend la composition cosmétique de base selon la revendication 9.

11. Utilisation à des fins non thérapeutiques de la composition cosmétique de base selon la revendication 9 ou de la préparation cosmétique selon la revendication 10 pour améliorer la régénération cutanée et/ou en tant que produit anti-âge.
